# EUROPÄISCHE PATENTSCHRIFT

(11) **EP 1 112 030 B1**
(45) Veröffentlichungstag und Bekanntmachung des Hinweises auf die Patenterteilung: **01.12.2004**
(21) Anmeldenummer: 99955693.9
(22) Anmeldetag: 14.09.1999
(51) Int. Cl.: A61B 17/22

(54) **VERFAHREN UND VORRICHTUNG ZUR VISUALISIERUNG DER AUSRICHTUNG VON THERAPEUTISCHEN SCHALLWELLEN AUF EINEN ZU BEHANDELNDEN BEREICH**
METHOD AND DEVICE FOR VISUALIZING THE ORIENTATION OF THERAPEUTIC SOUND WAVES IN AN AREA TO BE TREATED
PROCEDE ET DISPOSITIF POUR VISUALISER L'ORIENTATION D'ONDES ACOUSTIQUES THERAPEUTIQUES SUR UNE ZONE A TRAITER

(30) Priorität: 14.09.1998 DE 19841951
(43) Veröffentlichungstag der Anmeldung: 04.07.2001
(73) Patentinhaber: STORZ MEDICAL AG, CH-8280 Kreuzlingen (CH)
(72) Erfinder: HAGELAUER, Ulrich, CH-8598 Bottighofen (CH)
(74) Vertreter: Lohr, Georg, Dr.
(86) Internationale Anmeldenummer: PCT/DE1999/002913
(87) Internationale Veröffentlichungsnummer: WO 2000/015121

(56) Entgegenhaltungen:
- WO-A-91/07726
- DE-A- 3 811 872
- DE-A- 19 512 956
- DE-A- 19 515 748
- US-A- 4 829 986
- US-A- 5 526 814
- US-A- 5 687 737

## Beschreibung

### Technisches Gebiet

Die Erfindung bezieht sich auf ein Verfahren zur Visualisierung der Ausrichtung von therapeutischen Schallwellen auf einen zu behandelnden Bereich sowie auf eine Vorrichtung zur Durchführung dieses Verfahrens.

### Stand der Technik

Geräte für die Anwendung von therapeutischen Schallwellen sind allgemein bekannt:

Beispielsweise werden für die Lithotripsie intra- oder extrakorporal erzeugte pulsförmige Druck- bzw. Stoßwellen eingesetzt, während kontinuierliche Schallwellen z.B. zur Gewebeerwärmung verwendet werden.

In jedem Falle ist es erforderlich, den therapeutisch wirksamen Anteil des Schallfeldes - im folgenden verkürzt als "Schallwellenfokus" bezeichnet - auf die zu therapierende bzw. zu bearbeitende Behandlungsregion auszurichten. Das kann durch Bewegung der Schallquelle und/oder des Patienten sowie durch eine Beeinflussung der räumlichen Druckverteilung im Schallfeld und damit durch eine Fokusverlagerung relativ zur Schallquelle erfolgen.

Dieser Ausrichtvorgang, der im folgenden als "Positionierung" bezeichnet wird, soll aus einer Reihe von Gründen - beispielsweise aus Haftungsgründen - in der Regel nicht selbsttätig, sondern vom Anwender, also beispielsweise einem Arzt ausgeführt werden. Damit der Anwender die Positionierung durchführen kann, ist eine numerische oder graphische Anzeige erforderlich, die es dem Anwender erlaubt, den Schallwellenfokus auf die Behandlungsregion auszurichten.

Bei Geräten für die Lithotripsie mit extrakorporal erzeugten Druckwellen erfolgt die Anzeige z.B. durch Einblenden eine Fadenkreuzes in ein zweidimensionales Röntgen- oder Ultraschallbild:

Eine technisch einfache, für das Gerätekonzept aber evtl. ergonomisch und bezüglich des Bauaufwands nachteilige Lösung besteht darin, die Komponenten für die Bilderzeugung - also die Röntgenröhre/Bildverstärker bzw. den Ultraschallschwinger - mechanisch mit der Schallquelle zu verbinden, so dass eine feste Ortsbeziehung zwischen den bilderzeugenden Komponenten und der Ultraschallquelle besteht.

Bei neueren Entwicklungen wird vorgeschlagen, die Schallquelle beweglich anzuordnen und die räumliche Position der bilderzeugenden bzw. -gebenden Komponenten relativ zur Schallquelle über geeignete Messverfahren zu erfassen. Eine derartige Vorrichtung ist beispielsweise in der DE-A-195 12 956 beschrieben.

Da bei dieser Vorrichtung keine feste räumliche Zuordnung zwischen dem bilderzeugendem System und der Schallquelle mehr besteht, entfällt die Möglichkeit, feste Marken o.ä. einzublenden. Vielmehr muss die jetzt variable räumliche Lage des Schallwellenfokus relativ zur Behandlungsregion angezeigt oder dargestellt werden.

Technisch einfach ist es, diese Information numerisch als Abstand in einem räumlichen Koordinatensystem anzuzeigen. Die Positionierung wird damit aber zu einem zeitraubenden Vorgang, da der Anwender diese numerische Information in eine räumliche Bewegung umsetzen muss.

Eine weitere Positionierungs-Anzeige ist in der Abb. 4 der DE-A-195 12 956 beschrieben. Hierbei wird die Lage des Schallwellenfokus in einer Ebene und einer auf ihr senkrecht stehenden Achse dargestellt. Nachteilig ist hierbei, dass der Anwender stets zwei bewegliche Marken im Auge behalten muss, um die räumliche Lage zu beurteilen. Fehlinterpretationen werden bei dieser - nicht pseudo-dreidimensionalen - Anzeige zwar vermieden, aber die Umsetzung der Bildinformation erlaubt keine ausreichend rasche und ermüdungsfreie Positionierung.

Weiterhin ist es beispielsweise bei Computer-Spielen - nicht jedoch in medizinischen Anwendungen - Stand der Technik, stereoskopische bzw. pseudo-dreidimensionale Darstellungen mit Hilfe von zwei in einem Kopfhelm angebrachten, kleinen Bildschirmen zu erzeugen: die beiden Bildschirme führen die Bildinformation dem linken und rechten Auge getrennt zu ("head mounted displays").

Andere bekannte Anordnungen verwenden nur einen Bildschirm, auf dem die Bildinformation für das linke und rechtes Auge mit verschiedenen Techniken getrennt wird ("Shutter-Brillen", Polarisationsbrillen, Rot-Grün-Brillen).

Aus diesen Anwendungen außerhalb der Medizintechnik ist es bekannt, dass nicht alle Menschen in der Lage sind, aus Stereodisplays einen räumlichen Eindruck zu entwickeln. Aber auch dann, wenn ein räumlicher Eindruck entwickelt werden kann, treten bei längerer Anwendung Ermüdungserscheinungen auf, die u.a. zu Fehlinterpretationen führen können.

Für eine Lageanzeige in einer medizinischen Anwendung ist aber ein ermüdungsfreies Arbeiten und die Sicherheit gegen Fehlinterpretationen zu fordern. Schließlich hängt von der richtigen Umsetzung der Bildinformation der Erfolg und die Nebenwirkungsarmut eines therapeutischen Eingriffs ab.

Dokument US-A-5 526 814 beschreibt ein Verfahren zur Visualisierung der Ausrichtung von therapeutischen Schallwellen auf einen zu behandelnden Bereich unter Verwendung einer Anzeigeeinheit mit einem Bildschirm, auf dem die Ausrichtung der therapeutischen Schallwellen in Bezug auf den zu behandelnden Bereich symbolisiert dargestellt ist, wobei auf dem Bildschirm die Schallwellenquelle und der Raumbereich, durch den sich die Schallwellen ausbreiten, sowie der zu behandelnde Bereich perspektivisch durch Zuordnung körperlicher Figuren dargestellt sind, wobei sich die perspektivische Ansicht bei einer Änderung des Ortes bzw. der Ausrichtung der Schallwellenquelle und/oder des Behandlungsbereichs bewegungsrichtig ändert.

### Darstellung der Erfindung

Der Erfindung liegt die Aufgabe zugrunde, ein Verfahren zur Visualisierung der Ausrichtung von therapeutischen Schallwellen auf einen zu behandelnden Bereich sowie auf eine Vorrichtung zur Durchführung dieses Verfahrens anzugeben, bei dem die Positio-nierungs-Information durch eine einfach zu interpretierende graphischen Anzeige erfolgt.

Ein erfindungsgemäßes Verfahren, das diese Aufgabe löst, ist im Anspruch 1 angegeben. Weiterbildungen sind Gegenstand der Ansprüche 2 folgende. Eine Vorrichtung zur Durchführung des erfindungsgemäßen Verfahrens ist im Anspruch 19 angegeben.

Die Erfindung geht dabei von folgender Erkenntnis aus: Der erfindungsgemäße Lösungsansatz basiert auf Erkenntnissen des Entwerfens dreidimensionaler Körper auf Computern ("computer aided design", "CAD") und der darauf aufbauenden Anwendung der künstlichen Realität ("virtual reality", "VR"). Das Grundprinzip der VR-Darstellungstechniken liegt darin, dass sich die Perspektive der dargestellten.Körper mit dem Standort des Betrachters ändert. Im Gegensatz zur stereoskopischen Darstellung entsteht aus dem ruhenden Bild allein noch kein räumlicher Eindruck, da linkes und rechtes Auge die gleiche Information erhalten.

Erzeugt man hingegen eine Bildfolge, in der sich der Beobachterstandort kontinuierlich verändert, so entsteht der Eindruck, der Betrachter bewege sich durch eine Anordnung von Objekten (im folgenden Szene genannt).

Aus einer derartigen Bildfolge kann das menschliche Gehirn Schlüsse über die Größe und die relative Lage der dargestellten Objekte ziehen. Gleich verhält es sich, wenn man dem Betrachter erlaubt, Gegenstände in der Szene scheinbar zu ergreifen (virtuelle Hand) und sie relativ zueinander zu bewegen. Die Ähnlichkeit der realen mit der auf dem Bildschirm erzeugten Szene rufen beim Betrachter die Illusion hervor, er befinde sich tatsächlich in der Szene ("immersion", Immersion).

Der Grundgedanke der Erfindung ist es, solche Darstellungstechniken zur Positionierung des Schallwellenfokus auf eine Behandlungsregion zu nutzen.

Hierzu werden auf dem Bildschirm die Schallwellenquelle und der Raumbereich, durch den sich die Schallwellen ausbreiten, sowie der zu behandelnde Bereich (i. f. Behandlungsbereich) perspektivisch durch Zuordnung körperlicher Figuren dargestellt. Dabei ändert sich die perspektivische Ansicht bei einer Änderung des Ortes bzw. der Ausrichtung der Schallwellenquelle und/oder des Behandlungsbereichs und/oder des Standortes bzw. der Orientierung des Bildschirmes bewegungsrichtig.

Die Darstellung gibt die reale Lage der Schallquelle und der Behandlungsregion perspektivisch richtig wieder, um dem Anwender einen zutreffenden Eindruck über die Größe und die Abstände der Figuren zu geben. Zur Ausrichtung auf die Behandlungsregion bewegt sich die dem Schallwellenfokus entsprechende Figur in der dargestellten Szene genau so wie in der Realität. Der Anwender erhält damit den Eindruck, er befinde sich in der virtuellen Szene und führe dort die Schallquelle auf die Behandlungsregion zu (Immersion, virtuelle Hand).

Dabei ist es besonders bevorzugt, wenn sich die perspektivische Ansicht bei einer Änderung des Standortes einer Bedienungsperson entsprechend der Ansichtsänderung der Szene für die Bedienungsperson ändert bzw. manuell ändern lässt. Insbesondere werden die von einem Meßsystem, das die relative Lage zwischen Schallquelle und Behandlungsbereich bzw. Ortungseinrichtung erfasst, erhaltenen Signale über die räumliche Lage der Schallquelle nahezu in Echtzeit in eine äquivalente virtuelle Bewegung umgesetzt.

Die fokussierten therapeutischen Schallwellen werden als Kegel dargestellt, dessen Mantel in etwa dem Übergang zwischen fokussierter Welle und Randbeugungswelle entspricht. Bei einer Weiterbildung wird der Mantel des dargestellten Kegels mit einer derart durchbrochenen Oberfläche dargestellt, dass der Behandlungsbereich nicht verdeckt wird. Insbesondere kann der Mantel des dargestellten Kegel semitransparent dargestellt werden.

Der Behandlungsbereich wird in etwa in der Größe dargestellt, in der die Schallwellen eine therapeutische Wirkung entfalten. Insbesondere kann bei fokussierten Schallwellen der Behandlungsbereich als Kugel oder Ellipsoid dargestellt werden.

Eine besonders bevorzugte Ausgestaltung des erfindungsgemäßen Verfahrens weist eine Annäherungs-Anzeige auf, die die Annäherung bzw. Übereinstimmung des Bereichs, in dem die Schallwellen eine therapeutische bzw. bearbeitende Wirkung entfalten, an den Behandlungsbereich angibt. Durch eine zusätzliche numerische Anzeige oder andere geeignete Mittel erhält der Anwender eine quantitative Information, wie genau er die Positionierung durchgeführt hat.

Die Annäherungs-Anzeige kann akustisch - beispielsweise durch eine Veränderung der Tonhöhe (Tonfrequenz) und/oder der Ton-Wiederholfrequenz - erfolgen. Weiterhin kann die Annäherungs-Anzeige durch einen Umschlag der Farbe erfolgen, in der wenigstens eine der körperlichen Figuren dargestellt ist. Ferner ist es möglich, dass die Annäherungsanzeige numerisch erfolgt.

Um Ermüdungserscheinungen zu vermeiden, ist es bevorzugt, wenn die dargestellten Objekte Oberflächen besitzen ("Rendering"). Dabei ist es von Vorteil, wenn auf den dargestellten Oberflächen Lichteinfall und Lichtreflexion simuliert wird, wie sich beispielsweise bei einem endoskopischen oder chirurigischen Eingriff ergeben würden.

Hierzu kann eine virtuelle Beleuchtungsquelle an der Decke des Behandlungs- bzw. Bearbeitungsraums angeordnet sein. Alternativ oder zusätzlich kann eine virtuelle Beleuchtungsquelle im Zentrum des Behandlungsbereichs angeordnet sein. Eine Lichtquelle befindet sich über dem Anwender. Dies ist der gewohnte Lichteinfall, so dass heller erleuchtete Partien auf der Kegel- und Kugeloberfläche lagerichtig als oben eingeschätzt werden. Eine zweite Lichtquelle befindet sich in der Mitte der Behandlungsregion, so dass sich eine Aufhellung im Bereich der Kegelspitze ergibt, wenn man sich dem Ziel annähert. Damit werden unerwünscht starke Abschattungen bei Zielannäherung von oben vermieden.

Um die Orientierung des Anwenders zu erleichtern, ist es von Vorteil, wenn in das dargestellte Bild ortsfeste Teile des Behandlungs raums eingeblendet werden. Bei den ortsfesten Teilen kann es sich um eine Behandlungsliege und/oder eine Positioniereinrichtung für eine Ortungseinrichtung - beispielsweise einen Röntgen-C-Bogen oder eine Ultraschall-Ortungseinrichtung - handeln.

Für den Anwender es ferner von Vorteil, wenn eine Zoomfunktion realisiert ist, mit der die Umgebung des Behandlungsbereichs vergrößert dargestellt werden kann.

Weiterhin kann die Lage des Behandlungsbereichs in Abhängigkeit von dem Ausgangssignal einer Ortungseinrichtung dargestellt werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird. Es zeigen:
- Fig. 1: die Darstellung der Verteilung des Schalldrucks durch einen durchbrochenen Kegel,
- Fig. 2: eine Behandlungsszene, in die zur Erläuterung der Eingabemöglichkeit für den Benutzerstandort ein Röntgen C-Bogen eingeblendet ist,
- Fig. 3: eine Darstellung zur Erläuterung der Annäherung des therapeutischen Schallwellenfeldes an die Behandlungsregion, und
- Fig. 4: eine Vorrichtung zur Durchführung des erfindungsgemäßen Verfahrens.

### Beschreibung von Ausführungsbeispielen

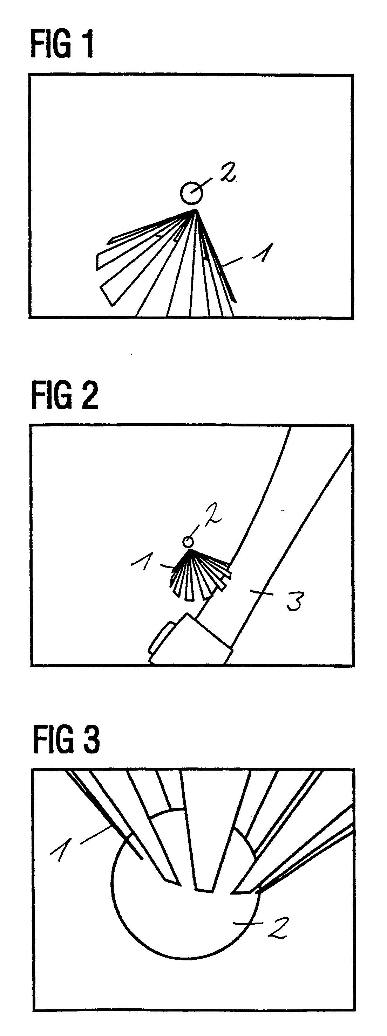

Fig. 1 zeigt, wie die Verteilung des Schalldrucks durch einen durchbrochenen Kegel 1 dargestellt wird. Mit 2 ist der Bereich bezeichnet, auf den das Schallwellenfeld ausgerichtet werden soll, und der mit dem in dieser Figur nicht dargestellten Schallwellenfokus "zur Deckung" gebracht werden soll.

Bei dieser Darstellung nutzt man die beim Anwender meist schon vorhandene Vorstellung des Eintrittskegels. Der Kegelmantel entspricht dem Übergang zwischen fokussierter Welle und Randbeugungswelle und stellt damit in erster Näherung den Raumbereich dar, innerhalb dem eine therapeutisch wirksame Schallenergie übertragen wird.

Die Unterbrechung des Kegelmantels dient dazu, eine Verdeckung der Behandlungsregion 2 zu vermeiden. Eine weitere Möglichkeit hierfür ist die halbtransparente Darstellung des Kegels ("rendering" im "transparent mode").

Der Schallwellenfokus kann nicht nur als Spitze des Kegels 1, sondern anschaulich auch in Form einer Kugel oder eines Rotationsellipsoids dargestellt werden, deren Größe der Größe des Schallwellenfokus entspricht. Somit ist der Anwender in der Lage, die erreichte Positioniergenauigkeit abzuschätzen. Bei einer Annäherung durchdringen Kegel 1, Kugel bzw. Ellipsoid (Schallwellenfokus) und Behandlungsregion 2 einander.

Dies ist in Fig. 3 dargestellt. Die entstehende Überschneidungsfigur erlaubt noch genauere Rückschlüsse auf die relative Lage von Kegel und Kugel im Vergleich zu der Situation, dass beide Objekte in einem Abstand voneinander dargestellt werden. Somit erhöht sich die Genauigkeit der Positionierung gerade auf den letzten Millimetern der Annäherung, was im Sinne der Anwendung erwünscht ist.

Fig. 2 erläutert eine Möglichkeit für die Darstellung des Benutzerstandorts. In Fig. 2 ist hierzu eine Behandlungsszene dargestellt, in die ein Röntgen C-Bogen 3 eingeblendet ist. Der C-Bogen 3 gibt dem Benutzer eine Orientierung über seinen Standort in der Szene. Mit Hilfe einer Maussteuerung, eines berührungssensitiven Bildschirms ("touch screen") o. dgl. kann die Szene gedreht werden. Durch Vergleich mit seinem wahren Standort wählt der Anwender die Perspektive, die seinem tatsächlichen Standort entspricht, so dass er in einer realen Szene arbeiten kann, ohne dass er zwischen Bildschirmdarstellung und tatsächlichem Behandlungsszenario "umdenken" müsste.

Weitere Möglichkeiten zur Verbesserung der räumlichen Orientierung bestehen in der (zusätzlichen oder alternativen) Einblendung einer Behandlungsliege oder anderer ortsfester Teile des Behandlungsplatzes.

Weiterhin ist es möglich, eine veränderliche Vergrößerung ("Zoom-Funktion") vorzusehen. Abhängig vom momentanen Abstand zwischen Kegelspitze und Kugelmitte wird die Vergrößerung verändert, so dass beim Anwender der Eindruck entsteht, er nähere sich selbst der Behandlungsregion. Einerseits wird dadurch der Effekt der Immersion verstärkt, andererseits wird die Feinabstimmung auf den letzten Millimetern der Annäherung verbessert.

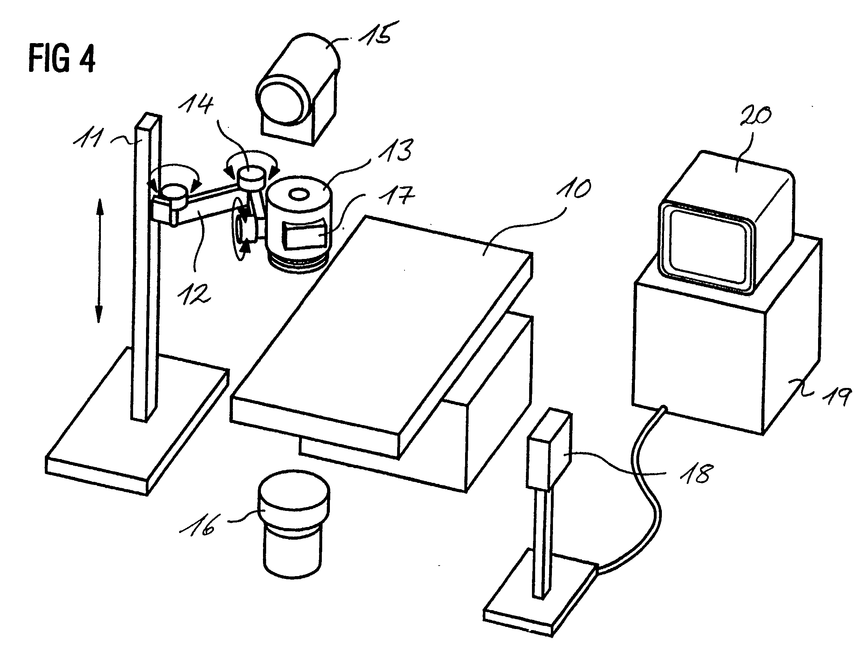

Fig. 4 zeigt eine typische Konfiguration für einen Behandlungsplatz mit Schallwellen, wie sie im Rahmen der Erfindung weitergebildet wird.

Der Behandlungsplatz weist eine Patientenliege 10 auf, die zur Positionierung eines nicht dargestellten Patienten in an sich bekannter Weise horizontal verschiebbar, höhenverstellbar und/oder drehbar ist. An einem Stativ 11 ist ein verstellbarer Haltearm 12 für eine therapeutische Schallquelle 13 angebracht. Die Schallquelle 13 kann dabei in an sich bekannter Weise ausgebildet sein und beispielsweise zur Zerstörung von Körperkonkrementen, zur Schmerzbehandlung, zur Behandlung des Herzens, zur Erwärmung von Körperregionen oder dgl. dienen. Ausdrücklich soll klargestellt werden, dass die vorstehende Aufzählung von Möglichkeiten für die Ausbildung der Schallquelle bildung der Schallquelle bzw. deren Anwendung nicht abschließend ist !

Mit 14 ist eine Bremse für die Bewegungen der Schallquelle 13 bezeichnet, die eine genaue Justierung der Schallquelle 13 relativ zum Patienten bzw. der Patientenliege 10 erlaubt. Die Schallquelle 13 kann damit in gewünschter Weise relativ zu einem auf der Patientenliege 10 liegenden Patienten von Hand- oder gegebenenfalls mit einer Servo-Verstellung - positioniert werden.

Eine Röntgenröhre 15 mit einem Bildverstärker 16, die beispielsweise an einem nicht dargestellten C-Bogen angebracht sein können, dient zur Erfassung des zu behandelnden Bereichs, also beispielweise eines Körperkonkrements in der Niere, der Blase oder den Harnwegen eines auf der Patientenliege 10 liegenden Patienten. Zusätzlich oder alternativ können auch andere Ortungssysteme, wie Ultraschall-Ortungssysteme vorgesehen sein.

An der Schallquelle 13 ist ein Sensor 17 angebracht, der die Erfassung der Position der Schallquelle 13 relativ zur Patientenliege 10 bzw. dem erkannten, zu behandelnden Bereich des Patienten mittels eines Positionserfassungssystems 18 erlaubt, dessen Ausgangssignal an eine Auswerte- und Steuereinheit, beispielsweise ein Computersystem 19 angelegt ist, das einen Bildschirm 20 aufweist, auf dem mittels des erfindungsgemäßen Verfahrens geeignete Anzeigen für eine Bedienungsperson, im Falle medizinischer Anwendungen also eines Arztes dargestellt werden. Nicht dargestellt sind geeignete Eingabemöglichkeiten, wie eine Tastatur, eine Maussteuerung, ein Touch-Screen oder eine Sprachsteuerung des Systems 19.

Während des Behandlungsvorgangs liefert das Positionserfassungssystem 18 kontinuierlich Daten über die Lage der Schallquelle 13 relativ zur Liege 10 an die Steuerund Auswerteeinheit bzw. das Computersystem 19.

Das Computerprogramm des Computersystems 19 beinhaltet schnelle Algorithmen zur Berechnung der Perspektive ("VR-Programme"), zur Erzeugung einer Oberfläche ("Rendering") und zur Berechnung der Lichtreflexion ("ray tracing"). Derartige Programme sind aus anderen - nichtmedizinischen - Anwendungen - beispielsweise CAD-Anwendungen - bekannt, so dass an dieser Stelle auf die Ausbildung derartiger Programme nicht näher eingegangen werden muss.

Anhand der Lagedaten wird die auf dem Bildschirm 20 dargestellte Szene mehrmals pro Sekunde aktualisiert, so dass sich die für eine Immersion erforderliche Echtzeit-Simulation ergibt.

Es können gleichzeitig auch andere Objekte wie z.B. der Bildverstärker 16 des Röntgengerätes oder ein Ultraschallschwinger erfasst und zur Verbesserung der Orientierung mit in der Szene dargestellt werden. Ebenso lassen sich mit dem gleichen Computersystem Bilder z.B. des Röntgensystems einlesen ("frame grabber") und aus der Lage des Bildverstärkers in zwei Projektionen die räumliche Lage der Behandlungsregion ermitteln, wie dies in der DE-A-195 12 956 beschrieben ist.

Quantitative Informationen über den Abstand der Kegelspitze zum Zentrum des Schallwellenfokus lassen sich auf verschiedene Art übermitteln. Zum Beispiel lässt sich eine numerische Anzeige des Abstands einblenden.

Eine andere Möglichkeit ist ein akustisches Signal, das bei größerer Entfernung mit niedriger Wiederholfrequenz ertönt. Mit abnehmendem Abstand wird die Wiederholfrequenz kontinuierlich erhöht. Bei Unterschreiten eines vorgegebenen Abstand wird die Tonhöhe angehoben.

Eine dritte Möglichkeit besteht in einem Farbumschlag von Kegel und/oder Kugel, der kontinuierlich oder bei Unterschreitung eines vorgegebenen Abstands erfolgt. Weiter kann vorgesehen werden, dass bei Unterschreiten eines vorgegebenen Zielabstands ein Signal erzeugt wird, das auf die Bremsvorrichtung 14 für die Bewegungen der Schallquelle 13 einwirkt.

Eine weiterführende Lösungsmöglichkeit besteht darin, das Lagesignal auf eine motorische Verstellung der Schallquelle 13 wirken zu lassen oder die räumlichen Druckverteilung der Schallquelle zu beeinflussen und somit die Positionierung zu automatisieren.

Eine weitere Ausgestaltung sieht vor, von einer dreidimensionalen Darstellung des Körperinneren auszugehen, wie sie u.a. mittels CT, NMR oder Ultraschall zu gewinnen ist. Die Kegelfigur kann dieser Darstellung überlagert werden, um die Lage des Schallwellenfeldes im Bezug auf anatomische Strukturen darzustellen. Dies kann die Therapie verbessern und Nebenwirkungen verringern, indem man beispielsweise vermeidet, dass sich gasgefüllte Hohlräume oder Knochenstrukturen im Schallfeld befinden.

Selbstverständlich kann das vorstehend beschriebene Verfahren außer im Bereich der Medizin auch in anderen Bereichen, wie der Materialbearbeitung eingesetzt werden, in denen ein fokussiertes Wellenfeld (beliebiger Wellen) eingesetzt wird, dessen Fokus mit einem Bearbeitungsbereich zur Deckung gebracht werden soll.

## Patentansprüche

1. Verfahren zur Visualisierung der Ausrichtung von fokussierten therapeutischen Schallwellen auf einen zu behandelnden Bereich unter Verwendung einer Anzeigeeinheit mit einem Bildschirm, auf dem die Ausrichtung der fokussierten therapeutischen Schallwellen in Bezug auf den zu behandelnden Bereich symbolisiert dargestellt wird, wobei
auf dem Bildschirm die Schallwellenquelle und der Raumbereich, durch den sich die Schallwellen ausbreiten, sowie der zu behandelnde Bereich, i.f. Behandlungsbereich, perspektivisch durch Zuordnung körperlicher Figuren dargestellt werden, und sich die perspektivische Ansicht bei einer Änderung des Ortes bzw. der Ausrichtung der Schallwellenquelle und/oder des Behandlungsbereichs und/oder des Standortes bzw. der Orientierung des Bildschirmes bewegungsrichtig ändert, wobei die fokussierten therapeutischen Schallwellen als Kegel dargestellt werden, dessen Mantel in etwa dem Übergang zwischen fokussierter Welle und Randbeugungswelle entspricht und wobei der Behandlungsbereich in etwa in der Größe dargestellt wird, in der die Schallwellen eine therapeutische Wirkung entfalten.

2. Verfahren nach Anspruch 1,
**dadurch gekennzeichnet, dass** sich die perspektivische Ansicht bei einer Änderung des Standortes einer Bedienungsperson entsprechend der Ansichtsänderung der Szene für die Bedienungsperson ändert bzw. manuell ändern lässt.

3. Verfahren nach einem der Ansprüche 1 oder 2,
**dadurch gekennzeichnet, dass** der Mantel des dargestellten Kegels mit einer derart durchbrochenen Oberfläche dargestellt wird, dass der Behandlungsbereich nicht verdeckt wird.

4. Verfahren nach einem der Ansprüche 1 oder 2,
**dadurch gekennzeichnet, dass** der Mantel des dargestellten Kegel semi-transparent dargestellt wird.

5. Verfahren nach einem der Ansprüche 1-4,
**dadurch gekennzeichnet, dass** bei fokussierten Schallwellen der Behandlungsbereich als Kugel oder Ellipsoid dargestellt wird.

6. Verfahren nach einem der Ansprüche 1 bis 5,
**dadurch gekennzeichnet, dass** eine Annäherungs-Anzeige vorgesehen ist, die die Annäherung bzw. Übereinstimmung des Bereichs, in dem die Schallwellen eine therapeutische Wirkung entfalten, an den Behandlungsbereich angibt.

7. Verfahren nach Anspruch 6,
**dadurch gekennzeichnet, dass** die Annäherungsanzeige akustisch erfolgt.

8. Verfahren nach Anspruch 7,
**dadurch gekennzeichnet, dass** die Annäherungsanzeige durch eine Veränderung der Tonhöhe (Tonfrequenz) und/oder der Ton-Wiederholfrequenz erfolgt.

9. Verfahren nach Anspruch 6,
**dadurch gekennzeichnet, dass** die Annäherungsanzeige durch einen Umschlag der Farbe erfolgt, in der wenigstens eine der körperlichen Figuren dargestellt ist.

10. Verfahren nach Anspruch 6,
**dadurch gekennzeichnet, dass** die Annäherungsanzeige numerisch erfolgt.

11. Verfahren nach einem der Ansprüche 1 bis 10,
**dadurch gekennzeichnet, dass** auf den dargestellten Oberflächen Lichteinfall und Lichtreflexion simuliert wird.

12. Verfahren nach Anspruch 11,
**dadurch gekennzeichnet, dass** eine virtuelle Beleuchtungsquelle an der Decke des Behandlungs raums angeordnet ist.

13. Verfahren nach Anspruch 11 oder 12,
**dadurch gekennzeichnet, dass** eine virtuelle Beleuchtungsquelle im Zentrum des Behandlungsbereichs angeordnet ist.

14. Verfahren nach einem der Ansprüche 1 bis 13,
**dadurch gekennzeichnet, dass** in das dargestellte Bild ortsfeste Teile des Behandlungs raums eingeblendet werden

15. Verfahren nach Anspruch 14,
**dadurch gekennzeichnet, dass** es sich bei den ortsfesten Teilen um eine Behandlungsliege und/oder eine Positioniereinrichtung für eine Ortungseinrichtung handelt.

16. Verfahren nach Anspruch 15,
**dadurch gekennzeichnet, dass** die Positioniereinrichtung ein Röntgen-C-Bogen ist.

17. Verfahren nach einem der Ansprüche 1 bis 16,
**dadurch gekennzeichnet, dass** eine Zoomfunktion realisiert ist, mit der die Umgebung des Behandlungsbereichs vergrößert dargestellt werden kann.

18. Verfahren nach einem der Ansprüche 1 bis 19,
**dadurch gekennzeichnet, dass** die Lage des Behandlungsbereichs in Abhängigkeit von dem Ausgangssignal einer Ortungseinrichtung dargestellt wird.

19. Vorrichtung zur Durchführung des Verfahrens nach einem der Ansprüche 1 bis 18,
**dadurch gekennzeichnet, dass** ein Positionserfassungssystem (17,18) vorgesehen ist, das die Lage der Schall quelle (13) relativ zu dem zu therapierenden Bereich erfasst, und dessen Ausgangssignal an eine Auswerte- und Steuereinheit (19) mit einem Bildschirm (20) angelegt ist, auf dem die Zuordnung zwischen Schallwellenfokus und zu behandelndem Bereich perspektivisch durch Zuordnung körperlicher Figuren dargestellt ist.

## Claims

1. Method for visually displaying the alignment of focussed therapeutic sound waves with a region to be treated, using a display unit having a monitor screen on which the alignment of the focussed therapeutic sound waves with respect to the region to be treated is symbolically represented, wherein
the sound wave source and the spatial region through which the sound waves spread out, and also the region to be treated, hereinafter termed a treatment region, are represented on the monitor screen in a perspective view of assigned three-dimensional figures, and the perspective view changes in true correspondence with movement upon a change of the position or alignment of the sound wave source, and/or the treatment region, and/or the site or orientation of the monitor screen, the focussed therapeutic sound waves being represented as cones having envelopes corresponding approximately to the transition between a focussed wave and a boundary diffracted wave, and the treatment region being represented as having approximately the size in which the sound waves are therapeutically effective.

2. Method according to claim 1,
**characterized in that** the perspective view changes or can be changed manually with a change of the location of an operating person to correspond with the operating person's changed view of the scene.

3. Method according to any one of claims 1 or 2,
**characterized in that** the envelope of the represented cone is displayed to have a surface which is broken so that the treatment region is not obscured.

4. Method according to any one of claims 1 or 2,
**characterized in that** the envelope of the represented cone is displayed as being semi-transparent.

5. Method according to any one of claims 1 to 4,
**characterized in that** with focussed sound waves the treatment region is displayed as a sphere or ellipsoid.

6. Method according to any one of claims 1 to 5,
**characterized in that** an approach indicator is provided which indicates that the range in which the sound waves are therapeutically effective is approaching or coinciding with the treatment region.

7. Method according to claim 6,
**characterized in that** an approach indication is made acoustically.

8. Method according to claim 7,
**characterized in that** an approach indication is made by a change of the pitch of a sound (audio frequency), and/or a sound repetition rate.

9. Method according to claim 6,
**characterized in that** an approach indication is made by a change of the colour in which at least one of the three-dimensional figures is displayed.

10. Method according to claim 6,
**characterized in that** an approach indication is made numerically.

11. Method according to any one of claims 1 to 10,
**characterized in that** light incidence and light reflection are simulated on the displayed surfaces.

12. Method according to claim 1,
**characterized in that** a virtual illumination source is disposed on the ceiling of the treatment room.

13. Method according to claim 1 or 2,
**characterized in that** a virtual illumination source is disposed at the centre of the treatment region.

14. Method according to any one of claims 1 to 13,
**characterized in that** stationary parts of the treatment room are faded into a displayed picture.

15. Method according to claim 14,
**characterized in that** the stationary parts are a treatment resting berth, and/or a positioning means for a locating means.

16. Method according to claim 15,
**characterized in that** the positioning means is an X-ray C arc.

17. Method according to any one of claims 1 to 16,
**characterized in that** a zoom function is implemented, by means of which the display of the surroundings of the treatment region can enlarged.

18. Method according to any one of claims 1 to 17,
**characterized in that** the position of the treatment region is displayed in dependence upon the output signal of a locating means.

19. A device for performing the method of any one of claims 1 to 18,
**characterized in that** a position detecting system (17, 18) is provided which detects the position of the sound source (13) relative to the region to be therapeutically treated, and the output signal thereof is applied to an evaluation and control unit (19) having a monitor screen (20) on which the positional relationship between sound wave focus and the region to be treated is displayed in a perspective view by means of the positional relationship of three-dimensional figures.

## Revendications

1. Procédé pour visualiser l'orientation d'ondes acoustiques thérapeutiques focalisées sur une zone à traiter au moyen d'une unité d'affichage comportant un écran sur lequel l'orientation des ondes acoustiques thérapeutiques focalisées par rapport à la zone à traiter est représentée de manière symbolique, dans lequel la source d'ondes acoustiques et la région de l'espace à travers laquelle les ondes acoustiques se propagent ainsi que la zone à traiter, ci-après appelée zone de traitement, sont représentées en perspective sur l'écran par affectation de figures tridimensionnelles ou volumiques et la vue en perspective se modifie dans le bon sens de déplacement en cas de changement du lieu ou de l'orientation de la source d'ondes acoustiques et/ou de la zone de traitement et/ou de la position ou de l'orientation de l'écran, dans lequel les ondes acoustiques thérapeutiques focalisées sont représentées sous la forme d'un cône dont l'enveloppe correspond à peu près à la transition entre onde focalisée et onde diffractée ou de courbure marginale et dans lequel la zone de traitement est représentée à peu près dans la grandeur dans laquelle les ondes acoustiques déploient une action thérapeutique.

2. Procédé selon la revendication 1,
**caractérisé par le fait que** la vue en perspective se modifie ou se laisse modifier manuellement conformément au changement de vue de la scène pour un opérateur lorsque cet opérateur change de position.

3. Procédé selon l'une des revendications 1 ou 2,
**caractérisé par le fait que** l'enveloppe du cône représenté est représentée par une surface ajourée de telle manière que la zone de traitement n'est pas masquée.

4. Procédé selon l'une des revendications 1 ou 2,
**caractérisé par le fait que** l'enveloppe du cône représenté est représentée de manière semi-transparente.

5. Procédé selon l'une des revendications 1 à 4,
**caractérisé par le fait que** lorsque les ondes acoustiques sont focalisées, la zone de traitement est représentée sous la forme d'une sphère ou d'un ellipsoïde.

6. Procédé selon l'une des revendications 1 à 5,
**caractérisé par le fait qu'**il est prévu une indication d'approche qui indique l'approche ou la coïncidence avec la zone de traitement de la zone dans laquelle les ondes acoustiques déploient une action thérapeutique.

7. Procédé selon la revendication 6,
**caractérisé par le fait que** l'indication d'approche est réalisée de manière acoustique.

8. Procédé selon la revendication 7,
**caractérisé par le fait que** l'indication d'approche est réalisée par modification de la hauteur du son (fréquence du son) et/ou de la fréquence de répétition du son.

9. Procédé selon la revendication 6,
**caractérisé par le fait que** l'indication d'approche est réalisée par un changement de la couleur dans laquelle au moins une des figures tridimensionnelles est représentée.

10. Procédé selon la revendication 6,
**caractérisé par le fait que** l'indication d'approche est réalisée de manière numérique.

11. Procédé selon l'une des revendications 1 à 10,
**caractérisé par le fait que** l'incidence et la réflexion de la lumière sont simulées sur la surface représentée.

12. Procédé selon la revendication 11,
**caractérisé par le fait qu'**une source lumineuse virtuelle est située au plafond du local de traitement.

13. Procédé selon la revendication 11 ou 12,
**caractérisé par le fait qu'**une source lumineuse virtuelle est située au centre de la zone de traitement.

14. Procédé selon l'une des revendications 1 à 13,
**caractérisé par le fait que** des éléments fixes du local de traitement sont ajoutés sur l'image représentée.

15. Procédé selon la revendication 14,
**caractérisé par le fait que** les éléments fixes en question sont un divan de traitement et/ou un dispositif de positionnement pour un dispositif de localisation.

16. Procédé selon la revendication 15,
**caractérisé par le fait que** le dispositif de positionnement est un appareil de radiographie à arceau en C.

17. Procédé selon l'une des revendications 1 à 16,
**caractérisé par le fait qu'**il est réalisé une fonction de zoom qui permet une représentation agrandie de l'environnement de la zone de traitement.

18. Procédé selon l'une des revendications 1 à 17,
**caractérisé par le fait que** la position de la zone de traitement est représentée en fonction du signal de sortie d'un dispositif de localisation.

19. Dispositif pour la réalisation du procédé selon l'une des revendications 1 à 18,
**caractérisé par le fait qu'**il est prévu un système de saisie de la position (17, 18) qui saisit la position de la source d'ondes acoustiques (13) par rapport à la zone à traiter et dont le signal de sortie est appliqué à une unité d'évaluation et de commande (19) comportant un écran (20) sur lequel l'association entre foyer des ondes acoustiques et zone à traiter est représentée en perspective par affectation de figures tridimensionnelles.
